(19) Europäisches Patentamt
European Patent Office
Office européen des brevets

(11) EP 2 376 218 B1

(12) **FASCICULE DE BREVET EUROPEEN**

(45) Date de publication et mention de la délivrance du brevet:
**04.11.2015 Bulletin 2015/45**

(21) Numéro de dépôt: **09803843.3**

(22) Date de dépôt: **11.12.2009**

(51) Int Cl.:
***B01J 13/04*** *(2006.01)*

(86) Numéro de dépôt international:
**PCT/FR2009/052500**

(87) Numéro de publication internationale:
**WO 2010/067037 (17.06.2010 Gazette 2010/24)**

# (54) PROCEDE DE PREPARATION DE NANOPARTICULES LIPIDIQUES

VERFAHREN ZUR HERSTELLUNG VON LIPIDNANOPARTIKELN

METHOD FOR PREPARING LIPID NANOPARTICLES

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **12.12.2008 FR 0858547**

(43) Date de publication de la demande:
**19.10.2011 Bulletin 2011/42**

(73) Titulaires:
- **UNIVERSITE D'ANGERS**
  **49000 Angers (FR)**
- **INSTITUT NATIONAL DE LA SANTE ET DE LA RECHERCHE MEDICALE (INSERM)**
  **75013 Paris (FR)**

(72) Inventeurs:
- **BENOIT, Jean-Pierre**
  **F-49100 Angers (FR)**
- **THOMAS, Olivier**
  **F-49100 Angers (FR)**
- **RAMADAN, Alyaa, Adel**
  **Zezenya ,Alexandrie (EG)**
- **SAULNIER, Patrick**
  **49330 Marigne (FR)**

(74) Mandataire: **Le Coupanec, Pascale A.M.P.**
**Cabinet Nony**
**3, rue de Penthièvre**
**75008 Paris (FR)**

(56) Documents cités:
**EP-A1- 1 955 695**
**WO-A1-01/64328**
**WO-A2-03/103822**
**WO-A2-2009/001019**
**WO-A2-2009/004214**
**FR-A1- 2 840 532**

- **ARNAUD BÉDUNEAU, E.A.: "Pegylated Nanocapsules Produced by an Organic Solvent-Free Method: Evaluation of their Stealth Properties" PHARMACEUTICAL RESEARCH, vol. 23, no. 9, 9 août 2006 (2006-08-09), pages 2190-2199, XP002539233**
- **BÉATRICE HEURTAULT, E.A.: "A Novel Phase Inversion-Based Process for the Preparation of Lipid Nanocarriers" PHARMACEUTICAL RESEARCH, vol. 19, no. 6, juin 2002 (2002-06), pages 875-880, XP002539234**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention vise à proposer un procédé utile pour préparer des nanocapsules lipidiques chargées en au moins un actif.
Des systèmes nanovésiculaires, de type nanocapsules ou nanogouttelettes dont la taille varie de 50 à 500 nanomètres et formés d'un coeur liquide ou semi-solide, enveloppé d'une membrane externe, sont déjà connus. Les constituants de leur membrane peuvent être synthétiques, par exemple de nature polymérique, protéique ou lipidique à l'image des liposomes. Notamment, les liposomes qui présentent une structure lamellaire formée d'un empilement de couches lipidiques séparées l'une de l'autre par des compartiments aqueux possèdent toujours un coeur aqueux.
Ces structures nanométriques ont également déjà été proposées à des fins d'encapsulation d'actifs soit dans leur coeur aqueux lorsque l'actif est hydrosoluble ou hydrodispersible, soit dans leur couche lipidique lorsque l'actif est liposoluble ou lipodispersible.
Par exemple, le brevet US 5,961,970 propose à titre de véhicule d'actifs, des émulsions huile-dans-eau à échelle submicronique, c'est-à-dire des miniémulsions dont les gouttelettes possèdent un noyau hydrophobe de nature lipidique et sont stabilisées en surface par des tensioactifs amphiphiles et/ou non ioniques à l'image des tensioactifs de type phospholipides. Ces gouttelettes sont ainsi maintenues en suspension dans une phase aqueuse. Ce type d'émulsion submicronique est obtenu à partir d'une émulsion de base en soumettant celle-ci à plusieurs cycles successifs d'homogénéisation sous fort cisaillement.
Quant au brevet US 5,576,016, il décrit des macroémulsions dont les gouttelettes sont formées d'un noyau lipidique solide et qui sont stabilisées par une enveloppe phospholipidique. Cette enveloppe phospholipidique possède une structure lamellaire formée d'une ou plusieurs couches de molécules phospholipides à l'image des liposomes. Un actif hautement hydrophobe peut être chargé au niveau du noyau et un actif hydrosoluble peut être en revanche incorporé dans les compartiments aqueux présents dans l'enveloppe phospholipidique.
WO 01/64328 décrit un procédé pour la préparation de nanocapsules à coeur lipidique liquide, écorce solide et chargées en un principe pharmaceutiquement actif à caractère hydrosoluble, ledit procédé comprenant les étapes consistant à

- disposer d'une émulsion, formulée par inversion de phase d'une émulsion et stabilisée par le Solutol HS 15. Cette émulsion contient aussi un tensio-actif lipophile, un triglycéride ou un ester d'acide gras et un principe pharmaceutiquement actif soluble ou dispersible en phase aqueuse.
- effectuer la trempe de l'émulsion obtenue, de manière à obtenir des nanocapsules.

Par ailleurs, les inventeurs ont également décrit dans le brevet EP 1 265 698 à titre de véhicule d'actifs liposolubles ou lipodispersibles, des nanocapsules à coeur liquide et écorce solide de nature lipidique et une technologie originale permettant d'y accéder. Plus précisément, ces nanocapsules chargées en actifs liposolubles ou lipodispersibles sont obtenues à partir d'une microémulsion, cette microémulsion étant préparée par la technique d'inversion de phase par effet thermique (émulsion PIT).
Le principe d'émulsification par inversion de phase en température (en anglais : Phase Inversion Temperature ou PIT) est bien connu de l'homme de l'art ; il a été décrit en 1968 par K. Shinoda («J. Chem. Soc. » Jpn., 1968, 89, 435). Il a été montré que cette technique d'émulsification permet d'obtenir des émulsions fines stables (K. Shinoda et H. Saito, « J. Colloid Interface Sci. », 1969, 30, 258).
Cette technique permet notamment d'accéder à une taille moyenne des globules constituant la phase huileuse variant de 0,1 à 4 $\mu$m (100 à 4000 nm).
Toutefois, lorsque ces nanocapsules sont destinées à encapsuler un actif, ce procédé impose de disposer dès sa première étape de la matière active que l'on souhaite encapsuler et donc d'effectuer l'intégralité du procédé en présence de cet actif. Or, cette obligation peut être parfois contraignante pour l'homme de l'art.
Par ailleurs, les nanocapsules obtenues n'y sont proposées qu'à des fins d'encapsulation, au sein de leur coeur huileux, d'actifs lipophiles ou lipodispersibles. Or, pour des raisons évidentes, il serait avantageux de tirer également profit de ces nanocapsules pour l'encapsulation d'actifs hydrosolubles ou hydrodispersibles.
Ainsi, l'homme de l'art ne dispose pas d'une méthode simple de mise en oeuvre et rapide pour accéder à des nanocapsules chargées en actifs, notamment en actifs à caractère hydrophile et, qui plus est, avec un rendement d'encapsulation intéressant.
La présente invention vise précisément à proposer un nouveau procédé permettant de répondre à ce besoin.
Plus précisément, la présente invention vise, selon un premier de ses aspects, un procédé utile pour la préparation de nanocapsules à coeur lipidique liquide, écorce solide et chargées en au moins un actif à caractère hydrophile, ledit procédé comprenant au moins les étapes consistant à :

i) disposer d'au moins une première microémulsion à caractère eau-dans-huile, stabilisée par au moins un tensioactif

lipophile et contenant au niveau de sa phase hydrophile au moins un actif à caractère hydrophile,
ii) disposer d'au moins une seconde microémulsion, distincte de la première microémulsion, formulée par inversion de phase d'une émulsion et stabilisée par au moins un tensioactif thermosensible, non ionique et hydrophile,
iii) ajouter ladite première microémulsion dans ladite seconde microémulsion, dans des conditions propices à la formation d'une nouvelle architecture microémulsion dans laquelle ledit actif hydrophile demeure présent au niveau de la phase hydrophile de la première microémulsion, et
iv) effectuer la trempe du mélange formé à l'étape précédente, de manière à obtenir des nanocapsules comprenant ledit actif hydrophile et étant formées d'un coeur lipidique, liquide à température ambiante et enrobé d'un film solide à température ambiante.

Selon un mode de réalisation particulier, l'actif hydrophile formulé selon le procédé selon l'invention, est à l'issue dudit procédé présent dans le coeur lipidique liquide desdites nanocapsules.
Selon un autre mode de réalisation particulier, l'invention concerne le procédé précité dans lequel la seconde microémulsion, avant mélange, est une microémulsion eau-dans-huile.
Au sens de la présente invention, le caractère hydrophile signifie que l'actif possède une affinité prépondérante pour l'eau ou un milieu aqueux.
Un composé hydrophile peut donc être en particulier un composé hydrosoluble, voire un composé hydrodispersible.
Au sens de la présente invention, on entend par phase hydrophile, voire polaire, un milieu totalement ou partiellement formé d'eau. Ainsi, il peut être formé en tout ou partie d'eau, en tout ou partie d'au moins un solvant polaire, ou d'un mélange eau/solvant polaire.
Selon un autre de ses aspects, la présente divulgation concerne des nanocapsules susceptibles d'être obtenues par ledit procédé selon la présente invention.
Elle vise également des nanocapsules à coeur lipidique liquide et écorce solide et chargées au sein de leur coeur lipidique liquide en au moins un actif à caractère hydrophile, en particulier hydrosoluble, ledit actif étant présent dans le coeur lipidique liquide sous la forme de microdomaines hydrophiles ou d'une microémulsion à caractère eau-dans-huile comprenant une phase huileuse stabilisée par au moins un tensioactif lipophile et une phase hydrophile incorporant ledit actif.
Elle vise en outre des compositions contenant de telles nanocapsules.
La présente invention résulte plus particulièrement de l'observation par les inventeurs que contre tout attente, une microémulsion obtenue par inversion de phase d'une émulsion, en particulier selon la technique inversion de phase en température, s'avère apte à interagir avec une microémulsion annexe de caractère eau-dans-huile et contenant au niveau de sa phase hydrophile ou encore polaire au moins un actif hydrophile, de manière à former une architecture de type microémulsion contenant ledit actif, au niveau de sa structure micellaire hydrophile interne.
De manière surprenante, l'interaction requise selon l'invention entre les deux microémulsions ne porte pas préjudice en terme de stabilité, aux microdomaines polaires ou encore structures micellaires contenant l'actif hydrophile présent dans la première microémulsion. Ces microdomaines se retrouvent internés dans des gouttelettes d'huiles dédiées à former le coeur lipidique liquide des nanocapsules lors de la trempe.

## MICROEMULSIONS

Tout d'abord, il importe de noter qu'une microémulsion est différente d'une mini- ou nano-émulsion et d'une macroémulsion notamment telles qu'illustrées dans les brevets US 5,961,971 et US 5,576,016, et qui sont des systèmes qualifiés de biphasiques. En effet, une microémulsion correspond à une structuration bicontinue de la matière sous forme de structures micellaires gonflées d'huile ou d'eau. Ces structures micellaires sont fortement imbriquées les unes par rapport aux autres et constituent ainsi un réseau tridimensionnel homogène cohésif et stabilisé. Ainsi, les microémulsions présentent des microdomaines pas nécessairement sphériques, de petites dimensions, typiquement de l'ordre de 10 à 50 nm, fluctuant dans le temps et dans l'espace.
En d'autres termes, les microémulsions ne sont pas des émulsions constituées de mini-gouttelettes. On ne peut y distinguer la phase dispersée de la phase continue. Enfin, contrairement aux macro/mini/nano-émulsions, elles sont thermodynamiquement stables. De telles microémulsions sont notamment décrites dans (Patel et al, « J. Agric. Food Chem. » 2006, 54, 7817-7824.
L'homme de l'art sait que la formulation des microémulsions se ramène au choix des valeurs de deux types de variables : les variables de composition et les variables de formulation physico-chimique.
On appelle « variables de composition » les proportions relatives des constituants principaux du système : le système tensioactif, la phase hydrophile ou polaire et la phase huileuse.
Les variables dites de formulation physico-chimique incluent pour leur part, tous les autres paramètres, physiques (température, pression) ou chimiques (nature des constituants principaux et des additifs) susceptibles d'influer

sur le système.

Pour ajuster ces paramètres nécessaires à la formation de la microémulsion attendue, une technique usuelle est d'élaborer un diagramme de phase correspondant.

Un tel diagramme peut être élaboré selon diverses méthodes dont la plus simple consistant à titrer un mélange de deux composants considérés avec le troisième composant. La figure 1, extraite de l'article de Prapaporn Boonme ; « Journal of Cosmetic Dennatology », 2007, 6, 233-228, illustre un exemple de diagramme de phase ternaire d'un système de microémulsion.

Une fois le diagramme de phases établi, la zone de microémulsion est identifiée et une microémulsion peut être facilement préparée en mélangeant simplement les composants requis dans un des rapports spécifiques existant dans cette zone de microémulsion.

Comme il ressort de ce diagramme, la microstructure d'une microémulsion, peut être décrite comme ayant un caractère huile-dans-eau (H/E), bicontinue, ou eau-dans-huile (E/H) selon la quantité relative de chacun des composés présents.

Il est également connu de caractériser ces systèmes par le rapport R de Winsor qui qualifie les interactions des molécules de tensioactifs localisées à l'interface avec les molécules voisines de la phase huileuse et de phase hydrophile selon l'équation :

$$- R = A_{SH}/A_{SE}$$

avec A désignant les interactions moléculaires par unité d'aire interfaciale et

E, H, S étant les indices se référant respectivement à la phase hydrophile, à la phase huileuse et au tensioactif.

Suivant que le rapport R est inférieur, supérieur, ou égal à l'unité, on obtient des diagrammes de phase caractéristiques appelés diagrammes de Winsor I, II ou III, tels que représentés sur la figure 2. Par ailleurs, les phases monophasiques claires de ces diagrammes sont qualifiées de microémulsions Winsor IV. Dans la plupart des cas, les limites de la zone de ce type de microémulsions sont signalées par la manifestation d'une solution trouble.

Ainsi, à partir d'une certaine concentration en tensioactifs, comprise entre 10 % et 50 %, la zone polyphasique disparaît et le tensioactif « co-solubilise » la phase hydrophile et la phase huileuse sous forme de structures plus ou moins organisées telles que des microémulsions ou des phases cristallines liquides (zone monophasique). Les lignes tracées dans la zone biphasique représentée sur cette figure 2 sont appelées lignes de conjugaison ou de partage.

L'inclinaison des lignes de conjugaison indique que la plus grande partie du tensioactif se trouve dans la phase aqueuse dans le cas de Winsor I et dans la phase huileuse dans le cas de Winsor II.

Dans le cas du diagramme Winsor III, il existe une zone inscrite dans un triangle ou le système se sépare en trois phases : une microémulsion contenant pratiquement tout le tensioactif et deux autres phases constituées dans la phase hydrophile et de la phase huileuse.

En revanche, à forte concentration en tensioactifs, on observe toujours une zone monophasique à laquelle on se réfère en parlant d'un comportement de type Winsor IV. En d'autres termes, une représentation de type Winsor IV correspond à la partie monophasique de l'un des diagrammes Winsor I, II et III de la figure 2.

## Microémulsion contenant l'actif hydrophile

Comme précisé précédemment, la première microémulsion peut comprendre au moins une phase grasse huileuse, au moins un tensioactif lipophile, une phase polaire ou hydrophile, et contenir au niveau de sa phase polaire ou hydrophile au moins un actif à caractère hydrophile, en particulier un actif hydrosoluble, voire hydrodispersible.

Selon un mode de réalisation préféré, cette microémulsion possède par ailleurs un comportement de type Winsor IV.

Avantageusement, cette microémulsion possède un caractère eau-dans-huile.

### a-Phase huileuse

La phase huileuse est formée d'au moins un corps gras liquide ou semi-liquide à température ambiante, et en particulier d'au moins un triglycéride, d'un ester d'acide gras ou d'un de leurs mélanges.

L'ester d'acide gras peut être plus particulièrement choisi parmi les esters d'acides gras en $C_4$ à $C_{18}$, en particulier en $C_8$ à $C_{12}$, et plus particulièrement en $C_8$ à $C_{10}$, et notamment parmi le tributyrine, le palmitate d'éthyle, l'oléate d'éthyle, le myristate d'éthyle, le myristate d'isopropyle, le myristate d'octydodécyle et leurs mélanges.

Les triglycérides mis en oeuvre peuvent être des triglycérides de synthèse ou des triglycérides d'origine naturelle. Les sources naturelles peuvent inclure les graisses animales ou les huiles végétales par exemple les huiles de

soja ou les sources en triglycérides à longue chaîne (LCT).

D'autres triglycérides d'intérêt sont composés principalement d'acides gras de longueurs moyennes encore appelés triglycérides à chaîne moyenne (MCT). Une huile à triglycérides à chaîne moyenne (MCT) est un triglycéride dans lequel la chaîne carbohydrate a de 8 à 12 atomes de carbone.

De telles huiles MCT sont disponibles commercialement.

A titre d'exemple de ces huiles MCT, on peut citer les TCR (nom commercial de la société industrielle des oléagineux, France, pour un mélange de triglycérides dans lequel environ 95 % des chaînes d'acides gras possèdent 8 ou 10 atomes de carbone) et le Myglyol® 812 (triglycéride commercialisé par la société Dynamit Nobel, Suède pour un mélange de triesters de glycérides d'acide caprylique et caprique).

Les motifs d'acides gras de ces triglycérides peuvent être insaturés, monoinsaturés ou polyinsaturés. Les mélanges de triglycérides ayant des motifs d'acides gras variables sont également acceptables.

Il est à noter que plus l'indice HLB du corps gras liquide ou semi-liquide est élevé, plus la température d'inversion de phase est élevée. En revanche, la valeur de l'indice HLB du corps gras ne semble pas avoir d'influence sur la taille des nanocapsules.

Ainsi, lorsque la taille des groupements terminaux des triglycérides augmente, leur indice HLB diminue et la température d'inversion de phase diminue.

L'indice HLB ou balance hydrophile-lipophile est tel que défini par C. Larpent dans le Traité K.342 des Editions TECHNIQUES DE L'INGENIEUR.

Convient tout particulièrement à l'invention, le triglycéride commercialisé sous le nom Labrafac WL 1349® ou Labrafac® CC.

<u>b-Phase hydrophile ou polaire</u>

En ce qui concerne la phase hydrophile ou polaire, elle peut être aqueuse ou non. Ainsi, elle peut être formée en tout ou partie d'eau, en tout ou partie d'au moins un solvant polaire, ou d'un mélange eau/solvant polaire.

Avantageusement, la phase hydrophile est constituée uniquement d'eau.

Selon un autre mode de réalisation, la phase hydrophile peut être exempte d'eau. En ce cas, elle peut être formée d'au moins un solvant hydrophile. Un tel solvant polaire est choisi pour sa capacité à solubiliser l'actif considéré, et au regard de l'application visée (chez l'homme ou l'animal) pour son innocuité. Ainsi, l'homme de l'art est à même de choisir un tel solvant.

De tels solvant polaires peuvent par exemple être choisis parmi les polypropylènes glycol, le propylène glycol, l'éthanol, l'isopropanol, le glycérol, le glycofurol, les polyéthylènes glycols de faible poids moléculaire, les polyols, et leurs mélanges. Par exemple, une telle phase hydrophile peut être constituée uniquement de propylène glycol [Patel et al, « J. Agric. Food Chem. » 2006, 54, 7817-7824].

Selon une autre variante de réalisation, la phase hydrophile peut être formée en tout ou partie d'eau.

Plus particulièrement, il peut s'agir d'un mélange eau/solvant(s) polaire(s), lesdits solvants polaires étant tels que définis précédemment.

Il est entendu que certains solvants polaires peuvent détenir par ailleurs des propriétés tensioactives. Il pourra être alors nécessaire de prendre en compte celles-ci lors de l'élaboration de la microémulsion. Le solvant considéré aura alors une double fonction, celle de solvant et de co-tensioactif, voire, dans certains cas, une fonction pour l'essentiel co-tensioactive.

Selon un mode de réalisation particulier, un tel mélange peut comprendre de 0,1 % à 50 % d'eau et de 50 % à 99,9 % d'au moins un solvant polaire.

Selon un autre mode de réalisation particulier, un tel mélange peut comprendre de 0,1 % à 50 % d'au moins un solvant polaire et de 50 % à 99,9 % d'eau.

Selon un autre mode de réalisation, un tel mélange peut être un mélange eau/propylène glycol, pouvant être formé de 10 % a 45 % d'eau, de préférence de 15 % à 35 %, tout particulièrement de 20 % à 30 % d'eau, voire d'environ 25 % d'eau, et de 50 % à 95 % de propylène glycol, de préférence de 55 % à 85 %, tout particulièrement de 70 % à 80 %, voire d'environ 75 % de propylène glycol.

Selon un autre mode de réalisation particulier, il s'agit d'un mélange équimolaire.

Selon un autre mode de réalisation, la phase hydrophile peut en outre comprendre un co-tensioactif, notamment à caractère hydrophile. De tels co-tensioactifs sont bien connus de l'Homme de l'art. Il peut par exemple s'agir d'alcools longs, du butanol, du pentanol, de l'hexanol, et leurs mélanges. D'autres exemples sont cités dans Patel *et al.* (*ibid*).

Il est entendu que cette première microémulsion peut comprendre en outre le cas échéant un actif dit lipophile en particulier liposoluble voire lipodispersible. Celui-ci sera alors interné au niveau de sa phase lipidique.

c- Tensioactifs

Comme précisé précédemment, la première microémulsion contenant au moins un actif hydrophile est stabilisée par au moins un tensioactif lipophile.

Pour des raisons évidentes, la nature du tensioactif nécessaire à la stabilisation de la microémulsion est directement liée à la nature chimique des phases hydrophile et lipophile mises en présence.

Ainsi, dans le cas d'une mise en présence d'une phase hydrophile formée pour l'essentiel d'un solvant à l'image du propylène glycol, la microémulsion correspondante peut être efficacement stabilisée par un unique tensioactif de nature lipophile.

En revanche, dans le cas où la phase hydrophile comprend de l'eau en association ou non avec un solvant polaire annexe, il peut être nécessaire d'associer au tensioactif lipophile un co-tensioactif.

Le tensioactif lipophile peut être solide ou non à température ambiante.

Le tensioactif lipophile mis en oeuvre selon la présente invention est plus particulièrement à base de phospholipides qui sont avantageux au regard de leur caractère biocompatible.

Parmi les phospholipides, les phosphatidylcholines (lécithine) sont tout particulièrement intéressants.

D'autres phospholipides peuvent être le phosphatidylglycérol, le phosphatidylinositol, la phosphatidylsérine, l'acide phosphatidique et la phosphatidyléthanolamine.

Les dérivés phospholipides peuvent être isolés à partir de sources naturelles ou préparés par synthèse.

A titre de produits commerciaux dérivant des phospholipides, on peut plus particulièrement citer :

- l'EPICURON 120® (Lukas Meyer, Allemagne) qui est un mélange d'environ 70 % de phosphatidylcholine, 12 % de phosphatidyléthanolamine, et environ 15 % d'autres phospholipides ;
- l'OVOTINE 160® (Lukas Meyer, Allemagne) qui est un mélange comprenant environ 60 % de phosphatidylcholine, 18 % de phosphatidyléthanolamine, et 12 % d'autres phospholipides,
- un mélange de phospholipides purifiés à l'image des produits Lipoïd E75 ou Lipoïds E-80® (Lipoïd, Allemagne) qui est un mélange de phospholipides comprenant environ 80 % en poids de phosphatidylcholine, 8 % en poids de phosphatidyléthanolamine, 3,6 % en poids de lipides non polaires et 2 % de sphingomyéline.

Selon un mode de réalisation préféré, le tensioactif lipophile est une lécithine dont la proportion en phosphatidylcholine varie de 40 à 80 % en poids.

Convient tout particulièrement comme source de phosphatidylcholine, le Lipoïd S75-3® (Lipoïd GmbH, Allemagne). Il s'agit de lécithine de soja. Cette dernière contient environ 69 % de phosphatidylcholine et 9 % de phosphatidyléthanolamine. Ce constituant est le seul constituant solide à 37 °C et à température ambiante dans la formulation.

On peut également utiliser le polyglycéryl-6-dioléate (Plurol®).

On peut aussi mettre en oeuvre, au titre de tensioactif liposoluble convenant à la présente invention, les sucroesters tels que les esters d'acide gras et de saccharose, les esters d'alkyl glucose, en particulier les esters gras de $C_1$-$C_6$ alkyl glucose polyoxyéthylénés, et leurs mélanges.

On peut plus particulièrement citer le mono- ou di-stéarate de sucrose et les polystéarates de sucrose ayant notamment une proportion élevée en monostéarate de sucrose.

Pour des raisons évidentes, les proportions en phase huileuse, phase hydrophile, et tensioactif sont à ajuster, au regard de la nature chimique des composés spécifiques mis en oeuvre, pour obtenir une microémulsion. Cet ajustement est réalisé *via* l'établissement d'un diagramme de phase, dont l'élaboration relève clairement des compétences de l'Homme de l'art. L'exemple 1, présenté ci-après, propose un mode de réalisation spécifique de formation d'une telle émulsion. Par ailleurs, les figures 3 et 4, qui rendent compte chacune d'un exemple de diagramme de phase, illustrent plusieurs exemples de premières microémulsions.

Selon un mode de réalisation, la première microémulsion est formée d'au moins un triglycéride d'acide gras, d'une lécithine, d'un dérivé 2-hydroxystéarate de polyéthylène glycol et d'un actif hydrophile.

La première microémulsion peut être réalisée à des températures variables, directement dépendantes du choix des composants requis pour former son architecture de microémulsion, à savoir la phase huileuse, la phase hydrophile ou polaire et les tensioactifs.

Il est à noter que la phase hydrophile contenant l'actif hydrophile, forme, au sein de la microémulsion, des microdomaines dont la taille peut être précisément ajustée à travers la température retenue pour former cette microémulsion. Cette taille est avantageusement inférieure à 50 nm.

d-Actif

Comme indiqué précédemment, le procédé est particulièrement avantageux pour internaliser, au sein des nanocapsules à coeur lipidique, un actif hydrophile, c'est-à-dire hydrosoluble ou hydrodispersible.

Selon une variante préférée de réalisation, cet actif est hydrosoluble.

Cet actif hydrophile est formulé selon l'invention via la première microémulsion eau-dans-huile qui l'intègre au niveau de sa structure micellaire hydrophile.

Autrement dit, l'actif à caractère hydrophile, voire hydrosoluble, est contenu dans des microdomaines de nature polaire ou hydrophile, stabilisés par au moins un tensioactif lipophile dans la phase huileuse.

Son incorporation préalable au sein de cette première microémulsion est réalisée par solubilisation dudit actif dans la phase hydrophile destinée à former la microémulsion.

Parallèlement, les tensioactifs dont au moins un tensioactif lipophile et la phase huileuse sont préalablement mélangés et une fois les tensioactifs solubilisés dans l'huile, les deux phases huileuse et aqueuse sont mises en présence.

L'ensemble est chauffé à une température propice à la réalisation de la microémulsion attendue.

Cette température varie de la température ambiante à 75 °C.

Ainsi, selon un mode de réalisation particulier, dans le procédé objet de la présente invention, la première microémulsion considérée pour le mélange est, lors du mélange, à une température variant de la température ambiante à 75 °C.

Cette température est généralement ajustée pour permettre un mélange homogène de l'ensemble des constituants. Cet ajustement est en particulier conseillé lorsque l'un des composés est solide à température ambiante à l'image par exemple de certains tensioactifs phospholipidiques. Ainsi, la mise en oeuvre de lipoïd® S75-3 à titre de tensioactif, pour former la microémulsion, impose de chauffer le mélange à une température supérieure à 72 °C, c'est-à-dire la température de fusion du lipoïd®.

La température du mélange peut être avantageusement maintenue jusqu'à l'introduction dans la seconde microémulsion.

Toutefois, l'ensemble peut le cas échéant être également placé à température ambiante ou à l'étuve à 37 °C jusqu'à obtention de la limpidité. Ce mode de réalisation s'avère tout particulièrement avantageux dans le cas d'actifs à caractère hydrophile thermosensibles.

Bien entendu, le procédé selon l'invention est également compatible avec la formulation d'actif(s) lipophile(s), c'est-à-dire liposoluble(s) ou lipodispersible(s).

Cet actif lipophile peut être introduit *via* la structure micellaire lipidique de la première ou seconde microémulsion.

Avantageusement, lorsqu'il est lipophile, l'actif est introduit *via* la seconde microémulsion.

Les actifs considérés selon l'invention peuvent être un composé pharmaceutiquement actif, cosmétiquement actif ou actif dans un domaine phytosanitaire ou alimentaire.

Selon un mode réalisation préféré, cet actif est un principe pharmaceutiquement actif.

Un tel actif peut être également de nature protéique ou peptidique. Il peut aussi s'agir d'acides nucléiques tels qu'un plasmide d'ADN ou un ARN d'interférence.

L'actif peut être aussi un radiopharmaceutique. Il peut également s'agir d'un gaz ou un fluide pouvant se transformer en gaz.

**<u>Deuxième microémulsion</u>**

Comme précisé précédemment, la seconde microémulsion est stabilisée par au moins un tensioactif hydrophile thermosensible.

Elle peut être à caractère eau-dans-huile (partie droite de la zone d'inversion de phase ou ZIP) ou huile-dans-eau (partie gauche de la ZIP).

Avantageusement, elle est de type eau-dans-huile.

Ainsi, selon un mode de réalisation particulier de la présente invention, la seconde microémulsion, avant mélange, est une microémulsion eau-dans-huile.

Les tensioactifs émulsionnants habituellement utilisés ont un HLB (HLB = Hydrophilic Lipophilic Balance) allant de 8 à 18. Ces émulsionnants, grâce à leur structure amphiphile, se placent à l'interface phase huileuse / phase aqueuse, et stabilisent ainsi les gouttelettes d'huile dispersées.

Ils sont généralement qualifiés de tensioactifs thermosensibles, hydrophiles et non ioniques.

Le choix d'un tel tensioactif s'avère particulièrement avantageux pour garantir l'opération d'inversion de phase par variation de température, conduisant à une architecture de microémulsion.

Ainsi, ce type de tensioactif, utilisé dans la seconde microémulsion selon l'invention, voit sa solubilité dans l'huile augmentée avec l'augmentation de la température. Le HLB de tels tensioactifs peut varier de 8 à 18, de préférence de 10 à 18, notamment de 10 à 16. Selon un mode de réalisation de l'invention, de tels tensioactifs peuvent être choisis parmi les alcools gras éthoxylés, les acides gras éthoxylés, les glycérides partiels d'acides gras éthoxylés, les triglycérides d'acides gras et leurs dérivés éthoxylés, et leurs mélanges.

Comme alcools gras éthoxylés, on peut citer par exemple les produits d'addition d'oxyde d'éthylène avec l'alcool laurylique, notamment ceux comportant de 9 à 50 groupes oxyéthylénés (Laureth-9 à Laureth-50 en noms CTFA) ; les

produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétostéarylique (mélange d'alcool cétylique et d'alcool stéarylique), notamment ceux comportant de 9 à 30 groupes oxyéthylénés (Ceteareth-9 à Ceteareth-30 en noms CTFA) ; les produits d'addition d'oxyde d'éthylène avec l'alcool cétylique, notamment ceux comportant de 9 à 30 groupes oxyéthylénés (Ceteth-9 à Ceteth-30 en noms CTFA) ; les produits d'addition d'oxyde d'éthylène avec l'alcool stéarylique, notamment ceux comportant de 9 à 30 groupes oxyéthylénés (Steareth-9 à Steareth-30 en noms CTFA) ; les produits d'addition d'oxyde d'éthylène avec l'alcool isostéarylique, notamment ceux comportant de 9 à 50 groupes oxyéthylénés (Isosteareth-9 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stéarate à PEG-50 stéarate) ; les palmito-stéarates de PEG-9 à PEG-50 ; les béhénates de PEG-9 à PEG-50 (en noms CTFA : PEG-9 béhénate à PEG-50 béhénate) ; et leurs mélanges.

On peut utiliser aussi des mélanges de ces dérivés oxyéthylénés d'alcools gras et d'acides gras.

Ces tensioactifs peuvent également être soit des composés naturels à l'image des phospholipides écholates ou des composés synthétiques tels que les polysorbates qui sont des esters d'acide gras de sorbitol polyéthoxylé (Tween®), les esters de polyéthylène glycol et de diglycéride provenant par exemple de l'huile de ricin (Crémophor®), des acides gras polyéthoxylés, par exemple de l'acide stéarique (Simulsol M-53®), des éthers d'alcool gras polyoxyéthylénés (Brij®), des éthers non phényles polyoxyéthylénés (Triton N®), des éthers hydroxylphényle polyoxyéthylénés (Triton X®).

Il peut plus particulièrement s'agir d'un 2-hydroxystéarate polyéthylène glycol et notamment celui commercialisé sous le nom Solutol® HS15 par la société BASF (Allemagne).

Selon un autre mode de réalisation, la seconde microémulsion considérée selon la présente invention peut, en outre, contenir un co-tensioactif. Plus particulièrement, ce co-tensioactif est à caractère lipophile.

Il est à noter que la taille des particules de la microémulsion diminue quand la proportion en agent tensio-actif hydrophile augmente et quand la proportion en agents tensioactifs (hydrophile et lipophile) augmente. En effet, l'agent tensio-actif hydrophile entraîne une diminution de la tension interfaciale et donc une stabilisation du système ce qui favorise l'obtention de petites particules.

Par ailleurs, la taille des particules augmente quand la proportion d'huile augmente.

Pour sa part, la phase aqueuse de la microémulsion peut également avantageusement contenir de 1 à 4 % d'un sel notamment inorganique comme par exemple le chlorure de sodium. En effet, la modification de la concentration en sel entraîne un déplacement de la zone d'inversion de phase. Ainsi, plus la concentration en sel augmente et plus la température d'inversion de phase est basse. Ce phénomène s'avère tout particulièrement intéressant pour l'encapsulation de principes actifs thermosensibles hydrophobes.

Selon un mode de réalisation particulier, la première microémulsion contenant l'actif selon l'invention peut également contenir un tel sel, de manière à maintenir, si nécessaire, la même osmolarité, lors de l'introduction de la première microémulsion dans la seconde.

Selon un mode de réalisation particulier, la seconde microémulsion contient avantageusement de 5 à 15 % de tensio-actif(s) hydrophile(s), de 5 à 15 % d'une phase huileuse, associé le cas échéant à 1 à 3 % de tensio-actif(s) lipophile(s) et de 64 à 89 % d'une phase aqueuse (les pourcentages sont exprimés en poids par rapport au poids total de la microémulsion).

Dans un mode de réalisation préféré, la phase grasse est un triglycéride d'acide gras, le tensio-actif lipophile est une lécithine et le tensio-actif hydrophile est le Solutol® HS15.

Une microémulsion convenant tout particulièrement à l'invention est accessible selon la technique d'inversion de phase, en particulier par une opération d'inversion de phase en température à partir d'une émulsion huile-dans-eau, stabilisée par le système tensioactif considéré pour la microémulsion. Cette technologie est plus particulièrement décrite dans le brevet EP 1 265 698 dont le contenu est intégré à la présente demande.

La seconde microémulsion est généralement obtenue en maintenant une agitation mécanique constante.

Ainsi, l'ensemble des constituants destinés à former la seconde microémulsion, est pesé dans un récipient. Le mélange est homogénéisé, par exemple au moyen d'un Rayneri 350 tours/rnin, et chauffé en augmentant progressivement la température au moyen d'un bain marie jusqu'à une température supérieure ou égale à la température d'inversion de phase $T_2$, c'est-à-dire jusqu'à l'obtention d'une phase blanche plus visqueuse qui indique l'obtention de l'émulsion inverse (E/H). Le chauffage est alors stoppé et l'agitation maintenue jusqu'à retour à la température ambiante, en passant par la température d'inversion de phase $T_1$, c'est-à-dire la température à laquelle se forme la microémulsion attendue, sous la forme d'une phase transparente ou translucide. Lorsque la température est redescendue en dessous de la zone d'inversion de Phase en Température ($T_1$), on obtient une émulsion huile-dans-eau.

Plus précisément, l'inversion de phase entre l'émulsion huile/eau et l'émulsion eau/huile se traduit par une diminution de la conductivité quand la température augmente jusqu'à ce qu'elle s'annule.

Ainsi, $T_1$ est une température à laquelle la conductivité est au moins égale à 90 - 95 % de la conductivité mesurée à 20 °C et $T_2$ est la température à laquelle la conductivité s'annule et l'émulsion eau dans huile se forme. La température moyenne de la zone d'inversion de phase correspond à la température d'inversion de phase (TIP).

Dans la zone de formation d'une microémulsion (mélange translucide), les interactions hydrophiles et hydrophobes sont équilibrées car la tendance du système tensioactif est de former aussi bien des micelles directes que des micelles inverses. Par chauffage au-delà de cette zone, il y a formation d'une émulsion E/H (mélange opaque blanc), car le tensioactif favorise la formation d'une émulsion eau dans huile. Puis lors du refroidissement en-dessous de la zone d'inversion de phase, l'émulsion devient une émulsion H/E.

Le cas échéant, il est possible de reproduire un ou plusieurs cycles de température autour de la zone d'inversion de phase entre $T_1$ et $T_2$, jusqu'à observer une suspension translucide, qui correspond à la formation d'une nouvelle microémulsion. On stabilise alors le système à une température qui correspond à la structuration du système en la nouvelle microémulsion attendue.

Selon une variante de réalisation, la seconde microémulsion de l'étape ii) peut subir préalablement à sa mise en contact avec la première microémulsion au moins une opération d'inversion de phase en température.

Plus particulièrement, cette opération d'inversion de phase comprend au moins les étapes consistant à :

- augmenter la température de la seconde microémulsion, jusqu'à une température $T_2$ supérieure à sa température d'inversion de phase (TIP) pour obtenir une émulsion eau-dans-huile suivie d'une diminution de la température jusqu'à une température $T_1$, $T_1$<TIP<$T_2$ pour obtenir de nouveau une émulsion huile-dans-eau,
- le cas échéant, effectuer un ou plusieurs cycles de température autour de la zone d'inversion de phase entre $T_1$ et $T_2$, et
- stabiliser ledit système à une température située dans ou au proche voisinage de l'inversion de phase pour former une nouvelle microémulsion obtenue par inversion de phase.

En ce qui concerne la phase huileuse susceptible d'être mise en oeuvre pour préparer cette seconde microémulsion, elle peut être formée à partir des huiles proposées ci-dessus pour la première microémulsion. Cette phase huileuse peut être identique ou non à celle formant la première microémulsion.

Avantageusement, dans le procédé selon l'invention, la phase huileuse de chacune des deux microémulsions comprend au moins un triglycéride, un ester d'acide gras ou l'un de leurs mélanges.

Avantageusement, la seconde microémulsion peut incorporer un principe actif de nature lipophile. Dans ce cas, les nanocapsules obtenues à l'issue du procédé selon l'invention incorporeront au niveau de leur coeur lipidique liquide au moins un actif hydrophile et au moins un actif liposoluble.

**Formation des nanocapsules**

Comme signalé précédemment, la formation des nanocapsules attendues impose d'introduire la première microémulsion telle que définie ci-dessus à la seconde microémulsion.

Pour des raisons évidentes, cette étape est réalisée dans des conditions compatibles avec le maintien des première et seconde microémulsions sous une forme stabilisée.

Plus précisément, la seconde microémulsion doit être à une température compatible avec le maintien de son architecture de microémulsion. Généralement cet état de stabilité est atteint à une température comprise dans sa zone d'inversion de phase, à savoir entre 60 °C et 90 °C, en particulier entre 65 °C et 85 °C, tout particulièrement entre 70 °C et 75 °C, voire de l'ordre de 72 °C,:

Ainsi, selon un mode de réalisation préféré, dans le procédé selon l'invention, la seconde microémulsion considérée pour le mélange est, lors du mélange, à une température variant entre 60 °C et 90 °C, de préférence entre 65 °C et 85 °C, tout particulièrement entre 70 °C et 75 °C, voire de 72 °C.

Pour des raisons de stabilité accrue, la seconde microémulsion est avantageusement maintenue à une température se situant dans la gamme haute de la zone d'inversion de phase, c'est-à-dire à une température supérieure à 75 °C, notamment entre 75 °C et 85 °C.

Selon la température d'inversion de phase retenue pour la seconde microémulsion, il peut être privilégié une architecture de microémulsion de type eau-dans-huile ou de type huile-dans-eau.

Pour ce qui est de la première microémulsion incorporant au moins un actif hydrophile, sa température peut également varier au regard de la température requise pour accéder à cette structure de microémulsion, compte-tenu des différents composants considérés pour la former.

En conséquence, les deux microémulsions peuvent être mises en présence, soit en étant toutes deux portées à la même température ou à des températures voisines, soit en étant respectivement à deux températures distinctes.

Au sens de la présente invention, on qualifie de « températures voisines », deux températures dont les valeurs ne diffèrent l'une de l'autre qu'au plus de 5 %.

Ainsi, selon un mode de réalisation particulier, dans le procédé objet de l'invention, les deux microémulsions mises en présence possèdent ou non la même température.

Selon la première alternative, la température considérée est généralement proche de, voire, celle requise pour accéder à un état de microémulsion pour la seconde microémulsion ou encore celle correspondant à une température d'inversion de phase de la seconde microémulsion.

Comme précisé ci-dessus, la première microémulsion peut être, pour sa part, réalisée sur une gamme de température plus large, directement dépendante du choix des composants requis pour la former.

Ainsi, cette première microémulsion peut être réalisée entre 20 et 80 °C, voire à 37 °C ou à la température ambiante.

Au sens de la présente invention, on entend par « température ambiante » une température de l'ordre de 25 °C.

Ce mode de réalisation à une température variant de la température ambiante à 37 °C, notamment à température ambiante, est tout particulièrement avantageux pour l'encapsulation d'actif thermosensible.

A titre d'exemple d'un mode de réalisation particulier d'un procédé selon l'invention on peut notamment citer celui consistant à mettre en présence une première microémulsion formée d'une phase huileuse contenant du Labrafac®, stabilisée par un tensioactif lipophile tel que le Lipoïd® et d'une phase hydrophile comportant uniquement du propylène glycol ou comprenant un mélange eau/propylène glycol dans un rapport 1:3, et une seconde microémulsion formée d'eau, Labrafac® et NaCl, stabilisée par un tensioactif hydrophile thermosensible tel que le Solutol® et dont la température retenue pour la former est généralement une température d'environ 70 °C, par exemple 72 °C. On pourra alors privilégier un chauffage de la première émulsion à une température pouvant être comprise entre 65 °C et 80 °C, notamment entre 70 °C et 75 °C, par exemple 72 °C. Les deux microémulsions seront dans ce cas portées à une même température ou des températures voisines lorsqu'elles seront mises en présence.

Quoiqu'il en soit, il est à noter que la mise en présence d'une première microémulsion contenant un actif dit thermosensible avec une seconde microémulsion, nécessitant pour sa part d'être par exemple à une température d'au moins 65 °C pour stabiliser son architecture de microémulsion, n'est pas préjudiciable à la stabilité dudit actif. En effet, la trempe effectuée immédiatement après l'étape de mélange, minimise efficacement le temps de contact de cet actif avec une telle température.

Le mélange des deux microémulsions considérées selon la présente invention s'effectue généralement sous agitation mécanique pour obtenir une homogénéisation des deux microémulsions.

Avantageusement, les deux microémulsions mélangées à l'étape iii) du procédé selon l'invention sont présentes dans un rapport pondéral propice à la stabilité de la seconde microémulsion.

Plus particulièrement, les deux microémulsions selon la présente invention sont mises en présence dans un rapport pondéral deuxième microémulsion/première microémulsion contenant l'actif, propice à la stabilité de la deuxième émulsion en particulier compris entre 2 et 20, de préférence entre 3 et 15, voire de l'ordre de 5.

La fusion des deux microémulsion ou encore leur homogénéisation peut être visualisée à l'oeil nu (solution limpide).

La nouvelle architecture microémulsion ainsi formée subit consécutivement une trempe selon l'invention.

Cette étape destinée à former les nanocapsules selon l'invention consiste en un refroidissement brusque de l'architecture microémulsion à une température propice à la solidification des films interfaciaux composant la microémulsion. Cette température est généralement très inférieure à la température d'inversion phase de la seconde microémulsion. Le refroidissement est avantageusement réalisé sous agitation magnétique.

Par exemple, on peut effectuer la trempe de ladite microémulsion chargée en un ou plusieurs actifs à une température au moins 30 °C inférieure à la TIP de la seconde émulsion (Température d'Inversion de Phase) au moment du trempage.

Cette trempe peut être effectuée en diluant le milieu de 3 à 10 fois à l'aide d'eau dé ionisée à 2 °C $^{+}/_{-}$ 1 °C jetée dans la microémulsion fine. Les nanocapsules obtenues sont alors maintenues sous agitation pendant 5 minutes.

L'organisation du système sous forme de nanocapsules après trempage se traduit visuellement par un changement d'aspect du système initial qui passe de blanc-transparent à blanc-translucide avec effet Tyndall (reflets bleutés). Ce changement se produit à une température inférieure à la TIP. Cette température est située généralement entre 6 et 15 °C en dessous de la TIP.

A l'issue du procédé selon l'invention, des nanocapsules chargés en au moins un actif selon la divulgation sont obtenues.

Au sens de l'invention, l'expression « chargées en un actif » signifie que les nanoparticules obtenues à l'issue du procédé selon l'invention, internalisent au moins un actif à caractère hydrophile selon l'invention au niveau de leur coeur lipidique liquide.

Comme précisé ci-dessus, le procédé considéré selon l'invention est particulièrement intéressant pour charger facilement et rapidement au sein d'un coeur lipidique d'une microémulsion, un actif hydrophile, processus qui n'est pas

accessible par des procédés conventionnels.

L'actif hydrophile est contenu dans le coeur lipidique sous la forme d'une structure micellaire hydrophile ou encore d'une microémulsion à caractère eau-dans-huile, comprenant une phase huileuse stabilisée par au moins un tenstioactif lipophile et une phase hydrophile incorporant ledit actif.

Les nanocapsules de la divulgation conviennent plus particulièrement pour l'administration des principes actifs suivants : les antiseptiques, les protéines dont celles impliquées dans le phénomène de coagulation, les peptides, les plasmides d'ADN, les ARNsi, les anti-infectieux tels que par exemple les antimycosiques, les antibiotiques, les anticancéreux, les immunosuppresseurs, les principes actifs destinés au Système Nerveux Central qui doivent passer la barrière hémato-encéphalique, tels que les antiparkinsoniens, les antalgiques et plus généralement les principes actifs pour traiter les maladies neurodégénératives.

L'encapsulation d'actif selon l'invention dans les nanocapsules selon la divulgation peut être mesurée par évaluation du rendement d'encapsulation.

Le rendement d'encapsulation RE (en %) des nanocapsules obtenues selon la présente invention correspond à la proportion d'actif présente dans les nanocapsules par rapport à l'actif total ajouté dans la formulation.

Plus précisément, ce rendement est calculé selon la formule suivante:

RE (%)= (quantité d'actif dans les nanocapsules x 100)/(quantité d'actif dans les nanocapsules + quantité d'actif libre).

Le dosage de l'actif selon l'invention internalisé peut être mesuré par spectrophotométrie, par exemple à l'aide d'un spectrophotomètre qui mesure la densité optique à une longueur d'onde de 633 nm.

Avantageusement, ce RE est d'au moins 10 %, en particulier compris entre 15 % et 35 %, plus particulièrement compris entre 35 % et 100 %, de préférence entre 35 % et 80 %, en particulier entre 40 % et 60 %, voire de 50 %.

Au sens de l'invention le terme nanocapsules est à distinguer de nanosphères. On entend par nanocapsules des particules constituées d'un coeur liquide ou semi-liquide à température ambiante, enrobé d'un film solide à température ambiante, par opposition à des nanosphères qui sont des particules matricielles, i.e. dont la totalité de la masse est solide. Ainsi, lorsque les nanosphères contiennent un principe pharmaceutiquement actif, celui-ci est finement dispersé dans la matrice solide.

Avantageusement, les nanocapsules obtenues selon l'invention possèdent une taille moyenne inférieure à 150 nm, de préférence inférieure à 100 nm, de préférence encore inférieure à 50 nm. Ces tailles peuvent être déterminées par spectroscopie à corrélation de photons, microscopie électronique à balayage, microscopie électronique à transmission en mode cryoscopique.

L'épaisseur du film solide est avantageusement comprise entre 2 à 10 nm. Elle est égale environ au dixième du diamètre des particules. Cette épaisseur peut être calculée par bilan de masse, ou visualisée par microscopie électronique à transmission par ombrage négatif ou alors par microscopie électronique à transmission en mode cryoscopique.

Compte-tenu de leur taille, les nanocapsules de la divulgation sont des particules lipidiques colloïdales.

L'indice de polydispersité des nanocapsules de l'invention est avantageusement compris entre 5 et 15 %. Cet indice est déterminé sur l'histogramme de taille obtenu par la méthode de spectroscopie à corrélation de photons.

Les nanocapsules sont chacune constituées d'un coeur essentiellement lipidique liquide ou semi-liquide à température ambiante, enrobé d'une écorce solide ou semi-solide à température ambiante.

Au sens de l'invention, l'expression « essentiellement lipidique » signifie que le noyau formant les nanocapsules selon l'invention est constitué à plus de 50 % en poids, en particulier à plus de 75 % en poids, notamment à plus de 80 % en poids, voire plus de 90 %, plus particulièrement plus de 95 % de son poids respectif, voire en totalité d'un ou plusieurs composés lipidiques (hydrophobes)

Au sens de l'invention, l'expression température ambiante désigne une température variant de 18 à 25 °C.

Ainsi, la présente divulgation vise également des nanocapsules à coeur lipidique liquide et écorce solide et chargées au sein de leur coeur lipidique en au moins un actif à caractère hydrophile, en particulier hydrosoluble, ledit actif y étant présent sous la forme d'une microémulsion à caractère eau dans huile, comprenant une phase huileuse stabilisée par au moins un tensioactif lipophile et une phase hydrophile incorporant ledit actif.

Selon un mode de réalisation préféré, ledit actif y est présent sous une forme distincte d'un système micellaire inverse.

La présente divulgation a également pour objet des compositions contenant lesdites nanocapsules.La présente invention est illustrée par les exemples suivants qui sont présentés à titre illustratif et non limitatif du domaine de l'invention.

Il est à noter que :

- PG = propylène glycol,

- IPP = Isopropyl palmitate.

**<u>Exemple 1 :</u> Préparation d'une première microémulsion selon l'invention encapsulant du fondaparinux**

Le fondaparinux est un pentasaccharide de formule générale $C_{31}H_{43}N_3Na_{10}O_{49}S_8$ qui suit :

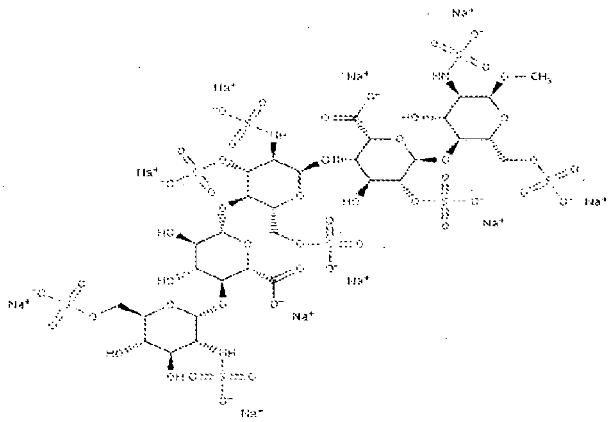

Sa solubilité dans l'eau est supérieure à 12,5 mg/mL (concentration du fondaparinux dans sa forme commerciale Arixtra®).

Pour la formulation de cette microémulsion, la phase hydrophile est composée uniquement de propylèneglycol (Patel et al., J. Agric. Food Chem. 2006, 54, 7817-7824). La microémulsion est également constituée par une phase huileuse (Labrafac® CC, mélange de triglycérides en $C_8$ et $C_{10}$) et un tensioactif liposoluble (Lipoïd® S75-3).

Le protocole général suivant est adopté pour élaborer le diagramme ternaire propylène glycol/Labrafac® CC/ Lipoïd® S75-3, conduisant à la caractérisation des zones monophasiques de type microémulsion, et qui est représenté en figure 3.

Le fondaparinux est solubilisé dans le propylène glycol à 75 °C. Une fois solubilisés, le Lipoïd® S75-3 et Labrafac® CC sont ajoutés et l'ensemble est chauffé à 75 °C pour obtenir une phase limpide qui est maintenue à cette température jusqu'à incorporation dans les nanocapsules lipidiques.

Une microémulsion est notamment formée en associant 185 mg de phase huileuse (Labrafac®), 40 mg de propylène glycol, 40 mg de tensioactif (Lipoïd® S75-3) et 500 µg de fondapirunux.

270 mg d'une microémulsion formée de 70 % en phase huileuse, 15 % en phase hydrophile et 15 % en tensioactif, sont obtenus.

**<u>Exemple</u>** <u>2 :</u> formulation de **la** microémulsion de l'exemple 1 à l'état de nanocapsules lipidiques

Dans un flacon de 20 mL, on mélange 504 mg de Solutol® HS 15, 44 mg de NaCl, 504 mg de Labrafac® CC et 1,538 g d'eau déminéralisée. Trois cycles de température entre 60 °C et 92 °C sont ensuite réalisés. Après la troisième montée en température, le milieu est refroidi à 72 °C. Il correspond à la seconde microémulsion considérée selon l'invention.

200 µL de la microémulsion obtenue selon l'exemple 1 (soit environ 190 mg), portée à cette même température, sont ajoutés sous agitation à cette seconde microémulsion, en une seule fois et rapidement.

Le mélange obtenu garde un aspect transparent.

Une trempe est ensuite réalisée à 70 °C, par ajout de 8 mL d'eau glacée.

La quantité du fondaparinux non encapsulé est déterminé par la méthode spectrophotométrique de complexation avec l'azure A. (Cadene, M. et al, « J. biol. Chem. » 1995, 270, 13204-13209).

Il est ainsi obtenu, une formulation de nanocapsules lipidiques encapsulant le fondaparinux à une concentration d'environ 100 µg de fondaparinux/g de nanocapsules lipidiques, soit un RE d'environ 35 à 40 %.

**<u>Exemple 3:</u> Formulation alternative de nanocapsules chargées en** fondaparinux

On prépare dans un premier temps une première microémulsion selon l'invention encapsulant du fondaparinux, par analogie avec le procédé de préparation décrit en exemple 1, mais dans lequel le fondaparinux est préalablement dissous dans 3,5 mg d'eau.

On ajoute ensuite 11,5 mg de propylène glycol, la phase polaire résultante étant ainsi constituée par un mélange

eau/propylène glycol 1:3. Une fois solubilisé, 37,5 mg de Lipoïd® S75-3 et 97,5 mg de Labrafac® CC sont également ajoutés au mélange précité. L'ensemble est chauffé à 75 °C. Le reste de la procédure est analogue à celle décrite en exemple 1. On obtient 150 mg de microémulsion et celle-ci, chauffée à 75 °C est totalement incorporée dans une seconde microémulsion préparée conformément au protocole décrit dans l'exemple 2 et, pour sa part, à une température de 72 °C.

Les paramètres étudiés pour la mise au point de cette première microémulsion sont les mêmes que ceux indiqués pour l'exemple 1 (figure 4).

Par analogie avec le mode opératoire décrit à l'exemple 2, on ajoute la première microémulsion contenant l'actif selon l'invention à la seconde microémulsion.

Il a été obtenu, de manière reproductible, une formulation de nanocapsules lipidiques encapsulant le fondaparinux à une concentration d'environ 50 μg de fondaparinux/g de nanocapsules lipidiques, soit un RE d'environ 15 à 25 %.

**Exemple** 4: Formulation **alternative** de nanocapsules chargées en **fondaparinux**

On prépare dans un premier temps une première microémulsion selon l'invention encapsulant du fondaparinux, par analogie avec le procédé de préparation décrit en exemple 1, mettant en oeuvre dans le cas présent 3 mg de fondaparinux, mais dans lequel le fondaparinux est préalablement dissous dans l'eau (15 mg).

On ajoute ensuite le Labrafac® CC (35 mg), l'Imwitor® 308 (35 mg) et le Span® 60 (15 mg) et on chauffe l'ensemble à 37° C pour obtenir une microémulsion limpide. Cette microémulsion est conservée à 37 °C jusqu'à son incorporation dans la seconde émulsion.

La seconde microémulsion est préparée selon le protocole décrit à l'exemple 2, à la différence qu'elle est obtenue à une température de 78 °C.

Par analogie avec le mode opératoire décrit à l'exemple 3, on ajoute 71 mg de la première microémulsion à une température de 37 °C contenant l'actif à 1 g de la seconde microémulsion à une température de 78 °C.

Il est ainsi obtenu, une formulation de nanocapsules lipidiques encapsulant le fondaparinux à une concentration d'environ 3000 μg de fondaparinux/g de nanocapsules lipidiques, soit un RE d'environ 100 %.

Ce dosage a été confirmé par un dosage chromogénique de l'activité anti XA.

**Exemple 5: Formulation alternative de nanocapsules chargées en fondaparinux**

On prépare dans un premier temps une première microémulsion selon l'invention encapsulant du fondaparinux, par analogie avec le procédé de préparation décrit en exemple 1 mettant en oeuvre dans le cas présent 1 mg de fondaparinux, mais dans lequel le fondaparinux est préalablement dissous dans l'eau (10 mg).

On ajoute ensuite le Labrafac® CC (37,6 mg), le Tween®80 (40 mg) et le Capmul® MCM (12,5 mg) et on laisse s'équilibrer l'ensemble à température ambiante pendant quelques heures pour obtenir une microémulsion limpide. Cette microémulsion est conservée à température ambiante jusqu'à son incorporation dans la seconde émulsion.

La seconde microémulsion est conforme à celle décrite à l'exemple 2, à la différence qu'elle est obtenue à une température de 80 °C.

Par analogie avec le mode opératoire décrit à l'exemple 3, on ajoute 83 mg de la première microémulsion contenant l'actif à la température ambiante à 1 g de la seconde microémulsion à une température de 72 °C.

Il est ainsi obtenu, une formulation de nanocapsules lipidiques encapsulant le fondaparinux à une concentration d'environ 350 μg de fondaparinux/g de nanocapsules lipidiques, soit un RE d'environ 35 %.

Ce mode de préparation mettant en oeuvre une première microémulsion contenant l'actif à température ambiante est particulièrement avantageux pour l'encapsulation d'actif à caractère hydrophile, voire hydrosoluble thermosensible.

**Exemple 6 : Formulation alternative de nanocapsules chargées en fondaparinux**

On prépare dans un premier temps une première microémulsion selon l'invention encapsulant du fondaparinux, par analogie avec le procédé de préparation décrit en exemple 1 mettant en oeuvre dans le cas présent 1 mg de fondaparinux, mais dans lequel le fondaparinux est préalablement dissous dans l'eau (20 mg).

On ajoute ensuite le Labrafac® CC (37,4 mg), le Tween80® (35 mg) et le Lipoïd® S75 (12,5 mg) et l'ensemble est chauffé à 75 °C pour obtenir une microémulsion limpide, analogue à celle décrite en exemple 1.

La seconde microémulsion est conforme à celle décrite à l'exemple 2, à la différence qu'elle est obtenue à une température de 80 °C.

Par analogie avec le mode opératoire décrit à l'exemple 3, on ajoute 20 mg de la première microémulsion contenant l'actif à 75 °C à 1 g de la seconde microémulsion à 72 °C.

Il est ainsi obtenu, une formulation de nanocapsules lipidiques encapsulant le fondaparinux à une concentration

d'environ 100 µg de fondaparinux/g de nanocapsules lipidiques, soit un RE d'environ 50 %.

## Revendications

**1.** Procédé utile pour la préparation de nanocapsules à coeur lipidique liquide, écorce solide et chargées en au moins un actif à caractère hydrophile, ledit procédé comprenant au moins les étapes consistant à :

i) disposer d'au moins une première microémulsion à caractère eau-dans-huile, stabilisée par au moins un tensioactif lipophile et contenant au niveau de sa phase hydrophile au moins un actif, à caractère hydrophile,
ii) disposer d'au moins une seconde microémulsion, distincte de la première microémulsion, formulée par inversion de phase d'une émulsion et stabilisée par au moins un tensioactif thermosensible, non ionique et hydrophile,
iii) ajouter ladite première microémulsion dans ladite seconde microémulsion, dans des conditions propices à la formation d'une nouvelle architecture microémulsion dans laquelle ledit actif hydrophile demeure présent au niveau de la phase hydrophile de la première microémulsion, et
iv) effectuer la trempe du mélange formé à l'étape précédente, de manière à obtenir des nanocapsules comprenant ledit actif hydrophile et étant formées d'un coeur lipidique, liquide à température ambiante et enrobé d'un film solide à température ambiante.

**2.** Procédé selon la revendication précédente dans lequel ledit actif hydrophile est, à l'issue dudit procédé, présent dans le coeur lipidique liquide desdites nanocapsules.

**3.** Procédé selon l'une des revendications 1 ou 2, dans lequel la seconde microémulsion, avant mélange, est une microémulsion eau-dans-huile.

**4.** Procédé selon l'une quelconque des revendications précédentes dans lequel la seconde microémulsion considérée pour le mélange est, lors du mélange, à une température variant entre 60 °C et 90 °C, de préférence entre 65 °C et 85 °C, tout particulièrement entre 70 °C et 75 °C, voire de 72 °C.

**5.** Procédé selon l'une quelconque des revendications précédentes dans lequel la première microémulsion considérée pour le mélange est, lors du mélange, à une température variant de la température ambiante à 75 °C.

**6.** Procédé selon l'une quelconque des revendications précédentes dans lequel les deux microémulsions mises en présence possèdent la même température.

**7.** Procédé selon l'une quelconque des revendications 1 à 5 dans lequel les deux microémulsions mises en présence possèdent des températures différentes.

**8.** Procédé selon l'une quelconque des revendications précédentes dans lequel la phase hydrophile de la première microémulsion contenant l'actif est formée en tout ou partie d'eau, en tout ou partie d'au moins un solvant polaire ou d'un mélange eau/solvant polaire.

**9.** Procédé selon la revendication précédente dans lequel le solvant polaire est choisi parmi les polypropylènes glycol, le propylène glycol, l'éthanol, l'isopropanol, le glycérol, le glycofurol, les polyéthylènes glycols de faible poids moléculaire, les polyols et leurs mélanges.

**10.** Procédé selon l'une quelconque des revendications précédentes dans lequel le tensioactif lipophile de la première microémulsion est à base de phospholipides.

**11.** Procédé selon l'une quelconque des revendications précédentes dans lequel la phase huileuse de chacune des deux microémulsions comprend au moins un triglycéride, un ester d'acide gras ou l'un de leurs mélanges.

**12.** Procédé selon l'une quelconque des revendications précédentes dans lequel la première microémulsion est formée d'au moins un triglycéride d'acide gras, d'une lécithine, d'un dérivé 2-hydroxystéarate de polyéthylène glycol et d'un actif hydrophile.

**13.** Procédé selon l'une quelconque des revendications précédentes dans lequel le tensioactif thermosensible, non

ionique et hydrophile de la seconde microémulsion est choisi parmi un le tensioactif hydrophile possédant un HLB allant de 10 à 18 notamment de 10 à 16.

**14.** Procédé selon l'une quelconque des revendications précédentes dans lequel le tensioactif hydrophile est choisi parmi les alcools gras éthoxylés, les acides gras éthoxylés, les glycérides partiels d'acides gras éthoxylés, les triglycérides d'acides gras et leurs dérivés éthoxylés et leurs mélanges.

**15.** Procédé selon l'une quelconque des revendications précédentes dans lequel la seconde microémulsion de l'étape ii) subit préalablement à sa mise en contact avec la première microémulsion au moins une opération d'inversion de phase en température.

**16.** Procédé selon la revendication précédente dans lequel ladite opération d'inversion de phase comprend au moins les étapes consistant à :

- augmenter la température de la seconde microémulsion, jusqu'à une température $T_2$ supérieure à sa température d'inversion de phase TIP pour obtenir une émulsion eau-dans-huile suivie d'une diminution de la température jusqu'à une température $T_1$, $T_1$<TIP<$T_2$ pour obtenir de nouveau une émulsion huile-dans-eau,
- le cas échéant, effectuer un ou plusieurs cycles de température autour de la zone d'inversion de phase entre $T_1$ et $T_2$, et
- stabiliser ledit système à une température située dans ou au proche voisinage de l'inversion de phase pour former une nouvelle microémulsion obtenue par inversion de phase.

**17.** Procédé selon l'une quelconque des revendications précédentes dans lequel les microémulsions de l'étape iii) sont mélangées dans un rapport pondéral propice à la stabilité de la seconde microémulsion.

**18.** Procédé selon la revendication précédente dans lequel le rapport pondéral deuxième microémulsion/première microémulsion contenant l'actif est compris entre 2 et 20, de préférence entre 3 et 15, voire de l'ordre de 5.

**19.** Procédé selon l'une quelconque des revendications précédentes dans lequel l'actif est un composé pharmaceutiquement actif, cosmétiquement actif ou actif dans un domaine phytosanitaire ou alimentaire.

## Patentansprüche

**1.** Verfahren zur Verwendung in der Herstellung von Nanokapseln mit einem flüssigen Lipidkern, einer festen Schale und bestückt mit wenigstens einem hydrophilen Wirkstoff, wobei in dem Verfahren Schritte ausgeführt werden, in denen:

i) wenigstens eine erste Mikroemulsion vom Wasser-in-Öl-Typ bereitgestellt wird, die durch wenigstens ein lipophiles Tensid stabilisiert ist und auf dem Niveau seiner hydrophilen Phase wenigstens einen hydrophilen Wirkstoff aufweist,
ii) wenigstens eine von der ersten Mikroemulsion verschiedene zweite Mikroemulsion bereitgestellt wird, die durch Inversion der Phase einer Emulsion gebildet ist und durch wenigstens ein wärmeempfindliches, nichtionisches und hydrophiles Tensidstabili-siert ist,
iii) die erste Mikroemulsion zu der zweiten Mikroemulsion zugegeben wird, und zwar unter günstigen Bedingungen für die Ausbildung einer neuen Mikroemulsions-Architektur, in der der hydrophilie Wirkstoff auf dem Niveau der hydrophile Phase der ersten Mikroemulsion vorhanden bleibt, und
iv) ein Aushärten der durch den vorangehenden Schritt gebildeten Mischung bewirkt wird, so dass Nanokapseln erhalten werden, die den hydrophilen Wirkstoff enthalten und mit einem Lipidkern ausgebindet sind, der bei Raumtemperatur flüssig ist und mit einem bei Raumtemperatur festen Film überzogen ist.

**2.** Verfahren nach dem vorstehenden Anspruch, in dem der hydrophile Wirkstoff am Ende des Verfahrens in dem flüssigen Lipidkern der Nanokapseln vorhanden ist.

**3.** Verfahren nach Anspruch 1 oder 2, in dem die zweite Mikroemulsion vor dem Vermischen eine Wasser-in-Öl-Mikroemulsion ist.

**4.** Verfahren nach einem der vorstehenden Ansprüche, in dem die zweite für die Mischung vorgesehene Mikroemulsion

beim Mischen eine Temperatur zwischen 60°C und 90°C, vorzugsweise zwischen 65°C und 85°C, besonders vorzugsweise zwischen 70°C und 75°C, insbesondere von 72°C, aufweist.

**5.** Verfahren nach einem der vorstehenden Ansprüche, in dem die für die Mischung vorgesehene erste Mikroemulsion beim Vermischen eine Temperatur aufweist, die zwischen Raumtemperatur und 75°C beträgt.

**6.** Verfahren nach einem der vorstehenden Ansprüche, in dem die zwei bereitgestellten Mikroemulsionen die gleiche Temperatur besitzen.

**7.** Verfahren nach einem der Ansprüche 1 bis 5, in dem die zwei bereitgestellten Mikroemulsionen verschiedene Temperaturen besitzen.

**8.** Verfahren nach einem der vorstehenden Ansprüche, in dem die hydrophile Phase der ersten Mikroemulsion mit dem Wirkstoff ganz oder zum Teil in Wasser, ganz oder zum Teil in wenigstens einem polaren Lösungsmittel oder in einer Mischung Wasser/polares Lösungsmittel gebildet ist.

**9.** Verfahren nach dem vorstehenden Anspruch, in dem das polare Lösungsmittel ausgewählt ist aus Polypropylenglykolen, Propylenglykol, Ethanol, Isopropanol, Glycerol, Glykofurol, Polyethylenglykol en mit geringem Molekulargewicht, Polyolen und deren Mischungen.

**10.** Verfahren nach einem der vorstehenden Ansprüche, in dem das lipophile Tensid der ersten Mikroemulsion eine Phospholipidbasis aufweist.

**11.** Verfahren nach einem der vorstehenden Ansprüche, in dem die Öl phase jeder der zwei Mikroemulsionen wenigstens ein Triglyzerid, ein Fettsäureester oder eine Mischung davon aufweist.

**12.** Verfahren nach einem der vorstehenden Ansprüche, in dem die erste Mikroemulson gebildet ist aus wenigstens einer Triglyzeridfettsäure, einem Lecithin, einem 2-Hydroxystearat-Derivat des Polyethylenglykols und einem hydrophilen Wirkstoff.

**13.** Verfahren nach einem der vorstehenden Ansprüche, in dem der wärmeempfindliche, nicht-ionische und hydrophileTensidwirkstoff der zweiten Mikroemulsion ausgewählt ist aus einem hydrophilen Tensidwirkstoff, der einen HLB-Wert von 10 bis 18, insbesondere von 10 bis 16, aufweist.

**14.** Verfahren nach einem der vorstehenden Ansprüche, in dem der hydrophilie Tensidwirkstoff ausgewählt ist aus Fettethoxylalkoholen, Fettsäureethoxylen, partiellen Glyzeriden von Fettsäureethoxylen, Fettsäuretriglyzeriden und deren Ethoxylderivaten und deren Mischungen.

**15.** Verfahren nach einem der vorstehenden Ansprüche, in dem die zweite Mikroemulsion des Schritts ii) vor seinem In-Kontakt-Bringen mit der ersten Mikroemulsion wenigstens einer Phaseninversionsoperation in der Temperatur unterzogen wird.

**16.** Verfahren nach dem vorstehenden Anspruch, in dem die Phaseni nversionsoperation wenigstens die folgenden Schritte aufweist:

Erhöhen der Temperatur der zweiten Mikroemulsion bis zu einer Temperatur $T_2$ oberhalb seiner Phaseninversionstemperatur (TIP), um eine Wasser-in-Öl-Emulsion zu erhalten, gefolgt von einer Verminderung der Temperatur bis zu einer Temperatur $T_1$, $T_1$<TIP<$T_2$, um erneut eine Öl-in-Wasser-Emulsion zu erhalten,
gegebenenfalls Durchführen eines oder mehrerer Temperaturzyklen, um die Phaseninversonszone zwischen $T_1$ und $T_2$, und
Stabilisierung des Systems bei einer Temperatur, die in oder benachbart zu der Phaseninversion liegt, um eine neue Mikroemulsion zu bilden, die durch die Phaseninversion erhalten wurde.

**17.** Verfahren nach einem der vorstehenden Ansprüche, in dem die Mikroemulsionen des Schritts iii) in einem Gewichtsverhältnis gemischt sind, das für die Stabilität der zwei - ten Mikroemulsion günstig ist.

**18.** Verfahren nach dem vorstehenden Anspruch, in dem das Gewichtsverhaltnis zweite Mikroemulsion/erste Mikroemulsion mit Wirkstoff zwischen 2 und 20, vorzugsweise zwi- schen 3 und 15, insbesondere i n der Größenordnung

von 5, liegt.

19. Verfahren nach einem der vorstehenden Ansprüche, in dem der Wirkstoff eine pharmazeutische Wirkstoffzusammensetzung, ein kosmetischer Wirkstoff oder ein Wirkstoff im Phytosanitär- oder Lebensmittel bereich ist.

## Claims

1. A useful method for preparing nanocapsules having a liquid lipid core and a solid shell and charged with at least one active agent having a hydrophilic character, said method comprising at least the steps consisting in:

   i) having at least a first microemulsion having a water-in-oil character, stabilized by at least one lipophilic surfactant and containing in its hydrophilic phase at least one active agent having a hydrophilic character;
   ii) having at least a second microemulsion, separate from the first microemulsion, formulated by phase inversion of an emulsion and stabilized by at least one heat-sensitive, nonionic hydrophilic surfactant;
   iii) adding said first microemulsion into said second microemulsion under conditions propitious for the formation of a novel microemulsion architecture in which said hydrophilic active agent remains present in the hydrophilic phase of the first microemulsion; and
   iv) chill-hardening the mixture formed in the previous step, so as to obtain nanocapsules comprising said hydrophilic active agent and being formed from a lipid core, which is liquid at room temperature, and encapsulated in a film which is solid at room temperature.

2. The method according to the preceding claim, in which said hydrophilic active agent is, after said method has been implemented, present in the liquid lipid core of said nanocapsules.

3. The method according to either of claims 1 or 2, in which the second microemulsion is, before mixing, a water-in-oil microemulsion.

4. The method according to any one of the preceding claims, in which the second microemulsion considered for the mixture is, during mixing, at a temperature varying between 60°C and 90°C, preferably between 65°C and 85°C, most particularly between 70°C and 75°C, for example a temperature of 72°C.

5. The method according to any one of the preceding claims, in which the first microemulsion considered for the mixture is, during mixing, at a temperature varying from room temperature to 75°C.

6. The method according to in any one of the preceding claims, in which the two microemulsions brought into contact have the same temperature.

7. The method according to any one of claims 1 to 5 in which the two microemulsions brought into contact have different temperatures.

8. The method according to any one of the preceding claims, in which the hydrophilic phase of the first microemulsion containing the active agent is formed entirely or partly from water, entirely or partly from at least one polar solvent or from a water/polar solvent mixture.

9. The method according to the preceding claim, in which the polar solvent is chosen from polypropylene glycols, propylene glycol, ethanol, isopropanol, glycerol, glycofurol, low-molecular weight polyethylene glycols, polyols and mixtures thereof.

10. The method according to any one of the preceding claims, in which the lipophilic surfactant of the first microemulsion is based on phospholipids.

11. The method according to any one of the preceding claims, in which the oily phase of each of the two microemulsions comprises at least a triglyceride, a fatty acid ester or one of the mixtures thereof.

12. The method according to any one of the preceding claims, in which the first microemulsion is formed from at least a fatty acid triglyceride, a lecithin, a 2-hydroxystearate derivative of polyethylene glycol and a hydrophilic active agent.

**13.** The method according to any one of the preceding claims, in which the heat-sensitive, nonionic hydrophilic surfactant of the second microemulsion is chosen from a hydrophilic surfactant having an HLB value ranging from 10 to 18, especially from 10 to 16.

**14.** The method according to any one of the preceding claims, in which the hydrophilic surfactant is chosen from ethoxylated fatty alcohols, ethoxylated fatty acids, partial glycerides of ethoxylated fatty acids, triglycerides of fatty acids and ethoxylated derivatives thereof and mixtures thereof.

**15.** The method according to any one of the preceding claims, in which the second microemulsion of step ii) is subjected, prior to being brought into contact with the first microemulsion, to at least one temperature-mediated phase inversion operation.

**16.** The method according to the preceding claim, in which said phase inversion operation comprises at least the steps consisting in:

- increasing the temperature of the second microemulsion up to a temperature $T_2$ above its phase inversion temperature (PIT) in order to obtain a water-in-oil emulsion, followed by reducing the temperature down to a temperature $T_1$, where $T_1 < PIT < T_2$ so as to obtain an oil-in-water emulsion again;
- where appropriate, carrying out one or more temperature cycles around the phase inversion zone between $T_1$ and $T_2$; and
- stabilizing said system at a temperature within or close to the phase inversion in order to form a new microemulsion obtained by phase inversion.

**17.** The method according to any one of the preceding claims, in which the microemulsions of step iii) are mixed in a weight ratio propitious for the stability of the microemulsion.

**18.** The method according to the preceding claim, in which the second microemulsion/first microemulsion containing the active agent weight ratio is between 2 and 20, preferably between 3 and 15, for example about 5.

**19.** The method according to any one of the preceding claims, in which the active agent is a pharmaceutically active compound, a cosmetically active compound or a compound that is active in the phytosanitory or food sector.

# FIGURE 1

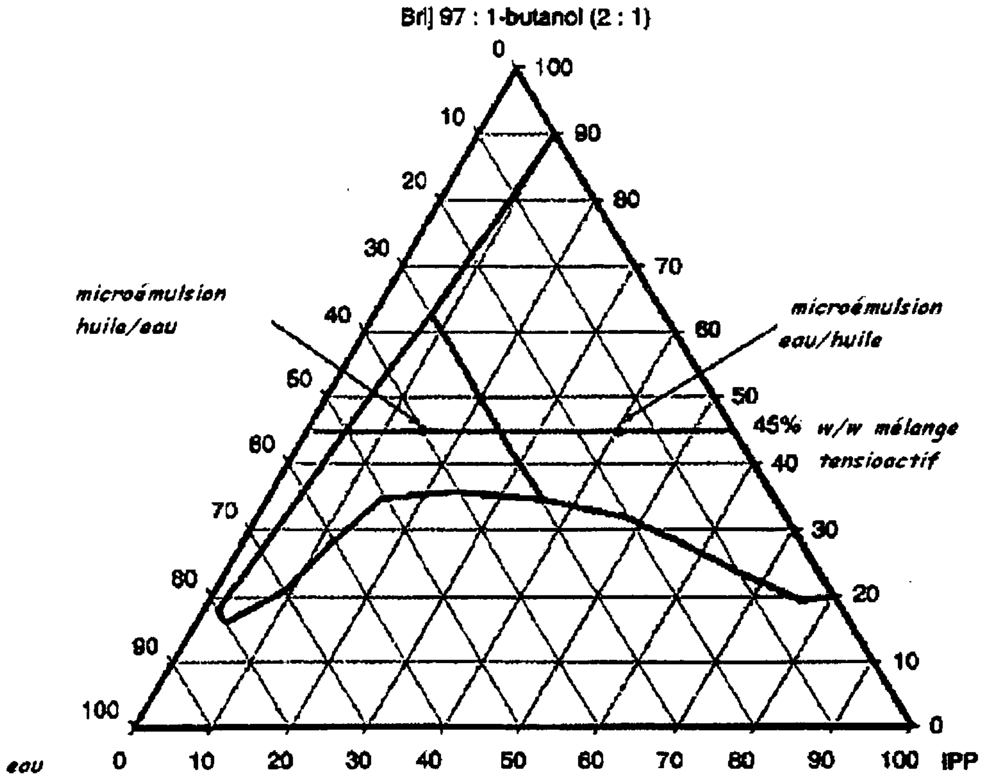
Brij 97 : 1-butanol (2 : 1)
0
100
10
90
20
80
30
70
40
60
50
45% w/w mélange tensioactif
40
30
20
10
0
microémulsion huile/eau
microémulsion eau/huile
eau
0 10 20 30 40 50 60 70 80 90 100
IPP

## FIGURE 2

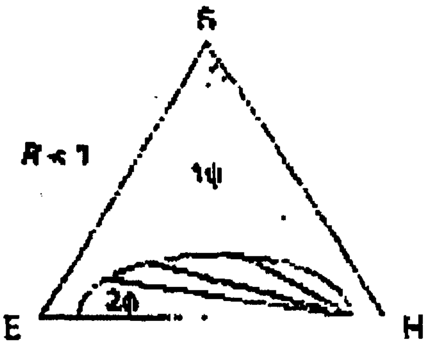


(a) diagramme de Winsor type I

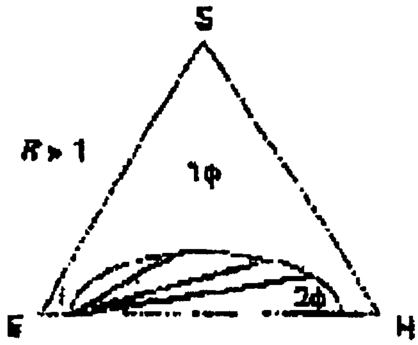


(b) diagramme de Winsor type II

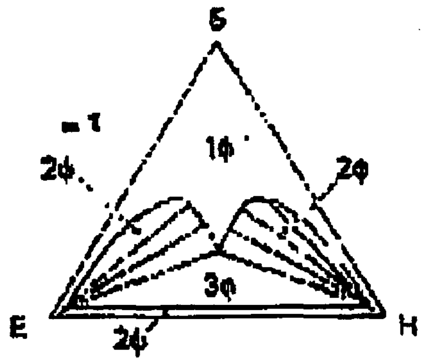


(c) diagramme de Winsor type III

une phase (1φ)
deux phases (2φ)
trois phases (3φ)

# FIGURE 3

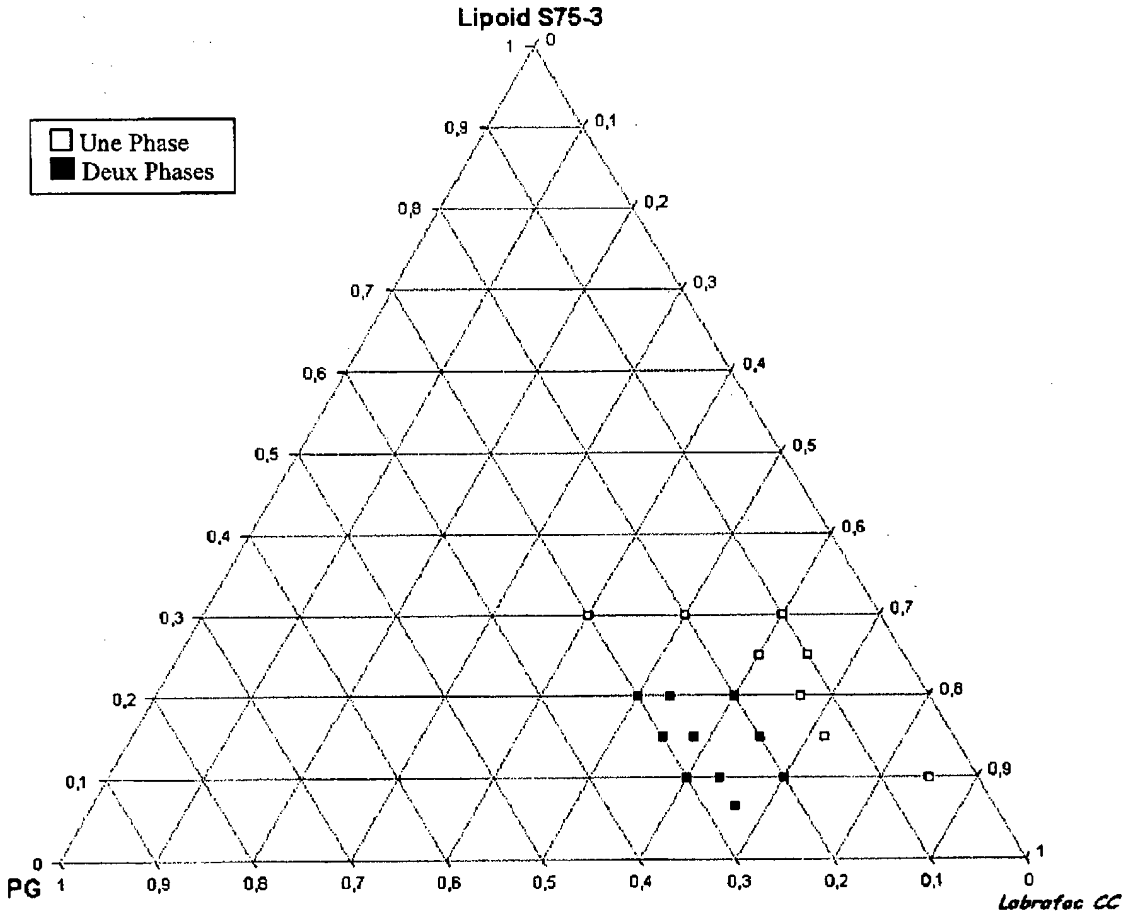
Lipoid S75-3
Une Phase
Deux Phases
1
0
0,9
0,1
0,8
0,2
0,7
0,3
0,6
0,4
0,5
0,5
0,4
0,6
0,3
0,7
0,2
0,8
0,1
0,9
0
1
PG
1
0,9
0,8
0,7
0,6
0,5
0,4
0,3
0,2
0,1
0
Labrafac CC

## FIGURE 4

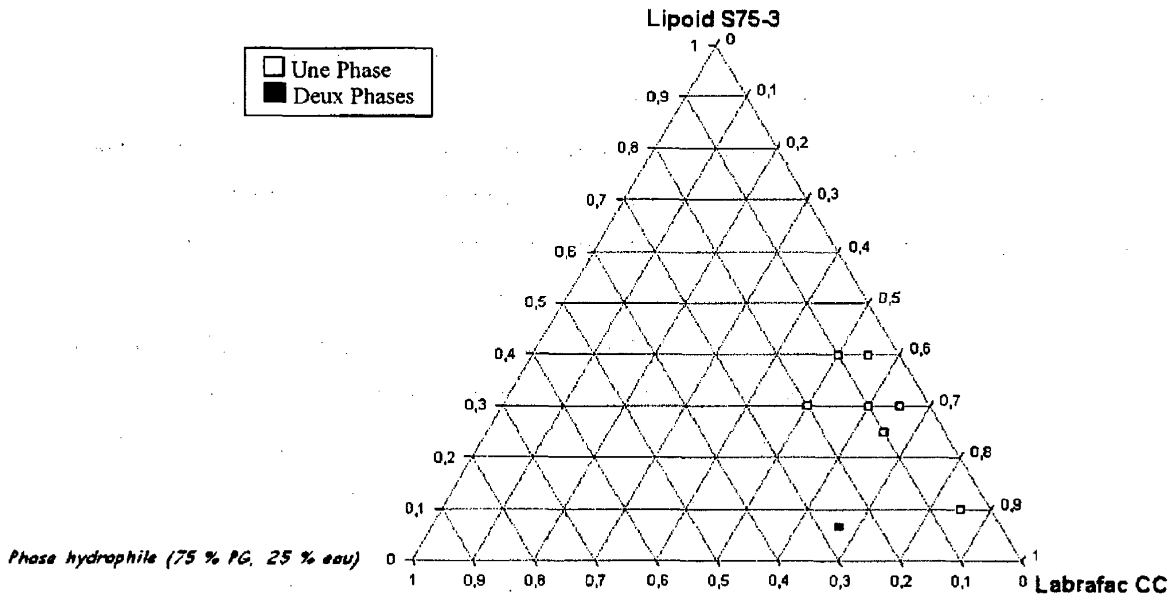
Lipoid S75-3
Une Phase
Deux Phases
Phase hydrophile (75 % PG, 25 % eau)
Labrafac CC
1
0
0,9
0,1
0,8
0,2
0,7
0,3
0,6
0,4
0,5
0,5
0,4
0,6
0,3
0,7
0,2
0,8
0,1
0,9
0
1
1
0,9
0,8
0,7
0,6
0,5
0,4
0,3
0,2
0,1
0

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5961970 A
- US 5576016 A
- WO 0164328 A
- EP 1265698 A
- US 5961971 A


### Littérature non-brevet citée dans la description


- **K. SHINODA.** *J. Chem. Soc.,* 1968, vol. 89, 435
- **K. SHINODA ; H. SAITO.** *J. Colloid Interface Sci.,* 1969, vol. 30, 258
- **PATEL et al.** *J. Agric. Food Chem.,* 2006, vol. 54, 7817-7824
- **PRAPAPORN BOONME.** *Journal of Cosmetic Dennatology,* 2007, vol. 6, 233-228
- **PATEL et al.** *J. Agric. Food Chem,* 2006, vol. 54, 7817-7824
- **CADENE, M. et al.** *J. biol. Chem,* 1995, vol. 270, 13204-13209